# EUROPÄISCHE PATENTANMELDUNG

(11) **EP 3 479 703 A1**
(43) Veröffentlichungstag der Anmeldung: **08.05.2019**
(21) Anmeldenummer: 17200455.8
(22) Anmeldetag: 07.11.2017
(51) Int. Cl.: A23L 33/175, A61K 31/198, A23L 33/11

(54) **NAHRUNGSERGÄNZUNGSMITTELPRÄPARAT**

(71) Anmelder: PM-International AG, 5445 Schengen (LU)
(72) Erfinder: Sorg, Rolf, 5444 Schengen (LU); Kühne, Tobias, 54497 Morbach (DE); Iken, Markus, 67105 Schifferstadt (DE); Lauinger, Christian, 76275 Ettlingen (DE); Messer, Wilhelm, 67058 Bad Dürkheim (DE)
(74) Vertreter: Zech, Stefan Markus

(57) **Zusammenfassung**

Die Erfindung betrifft ein Nahrungsergänzungsmittelpräparat, das pulverförmig, insbesondere gekapselt, vorliegt, umfassend
- eine L-Arginin-Komponente,
- eine Polyphenol-Komponente umfassend Polyphenole aus Äpfeln und/oder Trauben sowie
- eine Nitrit-Komponente gewonnen aus einem pflanzlichen Nitritlieferanten, insbesondere aus rotem Spinat und/oder Amaranth.

## Beschreibung

Die Erfindung betrifft ein Nahrungsergänzungsmittelpräparat, das pulverförmig, insbesondere gekapselt, vorliegt.

Der Einsatz von Nahrungsergänzungsmittelpräparaten beispielsweise im Sport oder zur Förderung der Gesundheit ist bestens bekannt.
Nahrungsergänzungsmittelpräparate sind dazu bestimmt, die allgemeine Ernährung bestehend beispielsweise aus kohlehydrat- und/oder eiweißhaltigen Lebensmitteln wie Fleisch, Gemüse, Obst oder dergleichen zu ergänzen. Im Gegensatz zu konventionellen Lebensmitteln und Getränken stellen Nahrungsergänzungsmittelpräparate üblicherweise ein Konzentrat von Nahrungsergänzungsstoffen oder sonstigen Stoffen mit ernährungsspezifischer, physiologischer Wirkung dar. Oft werden sie eingesetzt, um physiologische Vorgänge im Körper zu fördern und zu unterstützen.

Beispielsweise bildet der Körper im Endothelium körpereigenes NO (Stickstoffmonoxid) aus. Das körpereigene NO unterstützt diverse Vorgänge im Körper, fördert beispielsweise den Sauerstoffantransport an die Muskeln, insbesondere an die Zellen. Es wirkt der Ausbildung von Ablagerungen (Plaques) in den Blutgefäßen entgegen. Insgesamt hat es insoweit eine positive Auswirkung auf das Herz-Kreislauf-System und fördert den Blutantransport an die Organe, einschließlich der Sexualorgane.

Durch schlechte Ernährungsgewohnheiten, wenig Bewegung oder durch Rauchen, aber auch im Alter kann die körpereigene NO-Produktion nachlassen.

Die Aufgabe der vorliegenden Erfindung besteht darin, ein Nahrungsergänzungsmittelpräparat vorzuschlagen, das die Versorgung des Körpers mit NO (Stickstoffmonoxid) in mehrfacher Weise unterstützt.

Diese Aufgabe wird mit einem Nahrungsergänzungsmittelpräparat nach Anspruch 1 gelöst. Vorteilhafte Weiterbildungen sind in den Unteransprüchen angegeben.

Nach einer Kernüberlegung der vorliegenden Erfindung wird ein Nahrungsergänzungsmittelpräparat vorgeschlagen, das pulverförmig, insbesondere gekapselt, vorliegt und Folgendes umfasst:
- eine L-Arginin-Komponente,
- eine Polyphenol-Komponente umfassend Polyphenole aus Äpfeln und/oder Trauben sowie
- Eine Nitrit-Komponente gewonnen aus einem pflanzlichen Nitritlieferanten, insbesondere aus rotem Spinat und/oder Amaranth.

Bekanntermaßen katalysiert das Enzym eNOS die Bildung von NO (Stickstoffmonoxid) aus der Aminosäure L-Arginin. Die Zufuhr einer L-Arginin-Komponente kann insofern dazu beitragen, eine höhere Menge an NO zu synthesieren. Es hat sich weiterhin gezeigt, dass Polyphenole aus Äpfeln und/oder Trauben die Verfügbarkeit des Enzyms eNOS erheblich steigern können mit der Folge, dass eine deutlich erhöhte NO-Produktion erzielt werden kann mit den dann gegebenen bekannten positiven Auswirkungen wie Gefäßdilatation, Steigerung der körperlichen Leistungsfähigkeit etc. Schließlich kann die Zuführung einer Nitrit-Komponente die Verfügbarkeit von NO (Stickstoffmonoxid) begünstigen. Zu diesem Zweck sieht das Nahrungsergänzungsmittelpräparat nach der vorliegenden Erfindung eine Nitrit-Komponente gewonnen aus einem pflanzlichen Nitritlieferanten, insbesondere aus rotem Spinat und/oder Amaranth, vor.

Da in der körpereigenen Gewinnung von NO (Stickstoffmonoxid) aus Arginin auch L-Citrullin beteiligt ist, sieht das Nahrungsergänzungsmittelpräparat in einer vorteilhaften Weiterbildung eine L-Citrullin-Komponente zur Begünstigung der NO-Bildung vor.

Es hat sich auch gezeigt, dass körpereigenes Homocystein die NO-Produktion reduzieren kann. Durch Senkung des Homocysteinspiegels wird insofern die durch Homocystein gebremste NO-Produktion wieder erhöht. Insofern wird in einer bevorzugten Ausgestaltung vorgeschlagen, beim Nahrungsergänzungsmittelpräparat nach der vorliegenden Erfindung auch eine Homocysteinunterdrückungskomponente vorzusehen.

In einer konkreten Ausgestaltung ist eine Folsäurekomponente vorgesehen. Die Beigabe von Folsäure scheint die Verfügbarkeit des Enzyms eNOS zu steigern. Auch scheint die Folsäure den körpereigenen Homocysteinspiegel senken zu können.

Das Nahrungsergänzungsmittelpräparat kann weiterhin auch eine Vitamin B6- und/oder B12-Komponente enthalten. Vitamin B6 und/oder B12 können ebenfalls dazu beitragen, den Homocysteinspiegel zu senken.

Um den Antransport der jeweiligen ernährungsphysiologischen Komponente an dem Wirkort zu unterstützen, ist eine Bioverfügbarkeitskomponente zur Erhöhung der Bioverfügbarkeit, insbesondere der L-Arginin-Komponente und/oder der Polyphenol-Komponente und/oder der Nitrit-Komponente und/oder weiterer Komponenten mit ernährungsspezifischer, physiologischer Wirkung im vorliegenden Nahrungsergänzungsmittelpräparat in einer bevorzugten Ausgestaltung vorgesehen.

In einer bevorzugten Ausgestaltung ist diese Bioverfügbarkeitskomponente als Pfefferkomponente, insbesondere als Schwarze Pfefferkomponente, vorzugsweise als Extrakt aus Schwarzem Pfeffer, insbesondere Bioperin und/oder als eine Ingwer-Komponente, vorgesehen.

In einer weiterhin bevorzugten Ausgestaltung liegt das Nahrungsergänzungsmittelpräparat in gekapselter Form vor, wobei als Verkapselungsmaterial vorzugsweise Hydroxypropylmethylcellulose eingesetzt wird. In einer bevorzugten Ausgestaltung enthalten 100 g des Nahrungsergänzungsmittelpräparates:
- 25 g - 40 g, vorzugsweise etwa 32 g - 36 g, eines Trauben- und/oder Apfelextrakts als Polyphenol-Komponente und/oder
- 20 g - 40 g, vorzugsweise 28 g - 33 g, einer L-Arginin-Komponente und/oder
- 4 g - 12 g, vorzugsweise 5 g - 8 g, einer Nitrit-Komponente und/oder
- 3 g - 8 g, vorzugsweise 4 g - 6 g, einer L-Citrullin-Komponente und/oder
- 15 g - 23 g, vorzugsweise etwa 19 g, an Verkapselungsmaterial und/oder
- 40 µg - 100 µg, vorzugsweise 50 µg - 70 µg, an Vitamin B12 und/oder
- 20 mg - 50 mg, vorzugsweise zwischen 30 mg und 40 mg, an Vitamin B6 und/oder
- 3 mg - 6 mg, vorzugsweise etwa 5 mg, an Folsäure.

In einer weiterhin bevorzugten Ausgestaltung sind als weitere Komponenten
eine oder mehrere Anti-Caking-Komponenten, wie beispielsweise das Magnesiumsalz einer Fettsäure und/oder Talkum und/oder ein Verdickungsmittel, wie beispielsweise Glykosesyrup,
enthalten.

In einer bevorzugten Ausgestaltung umfasst die Tagesdosis 1 bis 3 Kapseln des erfindungsgemäßen Nahrungsergänzungsmittelpräparats, vorzugsweise 2 Kapseln, wobei die Tagesdosis vorsieht:
- 400 mg - 600 mg, vorzugsweise etwa 500 mg, der Polyphe3nol-Komponente und /oder
- 400 mg - 550 mg, vorzugsweise etwa 450 mg, der L-Arginin-Komponente und/oder
- 70 mg - 130 mg, vorzugsweise 100 mg, der Nitrit-Komponente und/oder
- 50 mg - 80 mg, vorzugsweise etwa 65 mg, der L-Citrullin-Komponente und/oder
- 50 µg - 90 µg, vorzugsweise etwa 70 µg, an Folsäure und/oder
- 0,7 µg - 1,2 µg, vorzugsweise etwa 0,9 µg, an Vitamin B12 und/oder
- 0,4 mg - 0,6 mg, vorzugsweise etwa 0,5 mg, an Vitamin B6.

In einem möglichen Ausführungsbeispiel der vorliegenden Erfindung kann das Nahrungsergänzungsmittelpräparat folgende Bestandteile enthalten:

| **Bestandteile** | **per 100 g [g]** | | **per Tagesdosis (2 Kapseln) [mg]** | |
|---|---|---|---|---|
| Trauben- und Apfelextrakt | 34,9 | | 5000 | |
| L-Arginin | 30,7 | | 440 | |
| Kapselschale (Hyd roxypropyl methylcellulose) | 19,0 | | 272 | |
| Quinoaextrakt (Amaranthus hypochondriacus) | 6,9 | | 100 | |
| L-Citrullin | 4,5 | | 65 | |
| Talkum (Antibackmittel) | 1,8 | | 26 | |
| Magnesiumsalz einer Fettsäure (Antibackmittel) | 0,8 | | 11,5 | |
| Glukosesyrup (Verdickungsmittel) | bis zu 100,000 hinzufügen | | 7,3 | |
| Geschmacksstoffe | 0,4 | | 6 | |
| Cyanocobalamin 0,1% auf Träger | 0,06 | = 61,2 mg Vitamin B12 | 0,875 | = 0,875 µg Vitamin B12 |
| Pyridoxinhydrochlorid | 0,05 | = 34,3 mg Vitamin B6 | 0,6 | = 0,49 mg Vitamin B6 |
| Folsäure | 0,005 | = 4897 µg Folsäure | 0,070 | = 70 µg Folsäure |

## Patentansprüche

1. Nahrungsergänzungsmittelpräparat, das pulverförmig, insbesondere gekapselt, vorliegt, umfassend
- eine L-Arginin-Komponente,
- eine Polyphenol-Komponente umfassend Polyphenole aus Äpfeln und/oder Trauben sowie
- eine Nitrit-Komponente gewonnen aus einem pflanzlichen Nitritlieferanten, insbesondere aus rotem Spinat und/oder Amaranth.

2. Nahrungsergänzungsmittelpräparat nach Anspruch 1, **gekennzeichnet durch**
- eine L-Citrullin-Komponente.

3. Nahrungsergänzungsmittelpräparat nach Anspruch 1 oder 2, **gekennzeichnet durch**
- eine Homocysteinunterdrückungskomponente.

4. Nahrungsergänzungsmittelpräparat nach einem der Ansprüche 1 bis 3, **gekennzeichnet durch**
- eine Folsäure-Komponente.

5. Nahrungsergänzungsmittelpräparat nach einem der Ansprüche 1 bis 4, **gekennzeichnet durch**
- eine Vitamin B6- und/oder B12-Komponente.

6. Nahrungsergänzungsmittelpräparat nach einem der Ansprüche 1 bis 5, **gekennzeichnet durch**
- eine Bioverfügbarkeits-Komponente zur Erhöhung der Bioverfügbarkeit der L-Arginin-Komponente und/oder der Polyphenol-Komponente und/oder der Nitrit-Komponente und/oder weiterer Komponenten mit ernährungsspezifischer physiologischer Wirkung in dem Nahrungsergänzungsmittelpräparat.

7. Nahrungsergänzungsmittelpräparat nach Anspruch 6, **dadurch gekennzeichnet, dass** die Bioverfügbarkeits-Komponente eine Pfefferkomponente, insbesondere eine schwarze Pfefferkomponente, vorzugsweise ein Extrakt aus schwarzem Pfeffer, insbesondere Bioperin, und/oder eine Ingwer-Komponente umfasst.

8. Nahrungsergänzungsmittelpräparat nach einem der Ansprüche 1 bis 7, **dadurch gekennzeichnet, dass** das Nahrungsergänzungsmittelpräparat in gekapselter Form vorliegt, wobei als Verkapselungsmaterial vorzugsweise Hydroxypropylmethylcellulose gewählt ist.

9. Nahrungsergänzungsmittelpräparat nach einem der Ansprüche 1 bis 8, **dadurch gekennzeichnet, dass** 100 g des Nahrungsergänzungsmittelpräparats enthalten:
- 25 g - 40 g, vorzugsweise etwa 32 g - 36 g, eines Trauben- und/oder Apfelextrakts als Polyphenol-Komponente und/oder
- 20 g - 40 g, vorzugsweise 28 g - 33 g, einer L-Arginin-Komponente und/oder
- 4 g - 12 g, vorzugsweise 5 g - 8 g, einer Nitrit-Komponente und/oder
- 3 g - 8 g, vorzugsweise 4 g - 6 g, einer L-Citrullin-Komponente und/oder
- 15 g - 23 g, vorzugsweise etwa 19 g, an Verkapselungsmaterial und/oder
- 40 µg 100 µg, vorzugsweise 50 µg - 70 µg, an Vitamin B12 und/oder
- 20 mg - 50 mg, vorzugsweise zwischen 30 mg und 40 mg, an Vitamin B6 und/oder
- 3 mg - 6 mg, vorzugsweise etwa 5 mg, an Folsäure.

10. Nahrungsergänzungsmittelpräparat, das als weitere Komponenten
eine oder mehrere Anti-Caking-Komponenten, wie beispielsweise das Magnesiumsalz einer Fettsäure und/oder Talkum und/oder ein Verdickungsmittel, wie beispielsweise Glykosesyrup,
enthalten.

11. Nahrungsergänzungsmittelpräparat nach einem der Ansprüche 1 bis 10, **dadurch gekennzeichnet, dass** als Tagesdosis 1 bis 3 Kapseln, vorzugsweise 2 Kapseln, vorgesehen sind, wobei die Tagesdosis
- 400 mg - 600 mg, vorzugsweise etwa 500 mg, der Polyphenol-Komponente und /oder
- 400 mg - 550 mg, vorzugsweise etwa 450 mg, der L-Arginin-Komponente und/oder
- 70 mg - 130 mg, vorzugsweise 100 mg, der Nitrit-Komponente und/oder
- 50 mg - 80 mg, vorzugsweise etwa 65 mg, der L-Citrullin-Komponente und/oder
- 50 µg - 90 ug, vorzugsweise etwa 70 µg, an Folsäure und/oder
- 0,7 µg - 1,2 µg, vorzugsweise etwa 0,9 µg, an Vitamin B12 und/oder
- 0,4 mg - 0,6 mg, vorzugsweise etwa 0,5 mg, an Vitamin B6 vorsieht.
